# EUROPEAN PATENT APPLICATION

(11) **EP 1 364 586 A1**
(43) Date of publication of application: **26.11.2003**
(21) Application number: 02011444.3
(22) Date of filing: 24.05.2002
(51) Int. Cl.: A23L 1/03, A61K 35/74, A23K 1/00, A61P 43/00, A61P 37/08, A61P 37/06

(54) **Probiotics and oral tolerance**

(71) Applicant: NESTEC S.A., 1800 Vevey (CH)
(72) Inventor: Pecquet, Sophia, 1012 Lausanne (CH); Prioult, Guénolée, Saint-Foy, QC (CA)
(74) Representative: Archambault, Jean

(57) **Abstract**

The invention refers to food products comprising at least a mean to promote the oral tolerance phenomenon (Lactobacillus paracasei), to food products comprising at least a mean to maintain the oral tolerance phenomenon (Bifidobacterium lactis), and to food products comprising means to promote and maintain the oral tolerance phenomenon (combination of Lactobacillus and Bifidocateria).

The food product is intended for infants, babies, children, and also for pets.

The invention refers to food products which are not intended for allergic populations.

## Description

### Background of the invention

Cow's milk allergy or milk hypersensitivity is very common in infants and usually disappears by the age of two or three years, but may occasionally be lifelong. It is the most common disease in infants, with an incidence of 0.5 to 3% in full term infants and 3 to 5% in preterm infants. This allergy can cause rash, hives, redness around the mouth, runny nose, sneezing, colic, diarrhoea, vomiting, anaphylaxis, or more generally digestive troubles. It could also be associated in some cases of infant sudden death.

Milk hypersensitivity should be differentiated from lactose intolerance, which is an intolerance to milk as a result of congenital deficiency of the lactase enzyme.

Cow's milk allergy is caused, in most cases, by the α-lactoglobulin and the β-lactoglobulin allergens. It can also be caused by casein and/or albumin, which are potentially allergenic lactic proteins also present in cow's milk. The allergy, when developed, is caused by an hypersensitivity reaction of the immune system to the above-mentioned proteins. In the first step of the process (sensitisation), the immune system recognises the protein as an antigen when it enters the body, and this generates an immune response consisting of specific antibodies or specifically sensitised T lymphocytes. In the second step, if the antibody is an IgE antibody, it will respond to the presence of the allergen by generating an inflammatory reaction, which is the allergy.
The mechanism of this type of allergy can be explained as follows: the IgE antibodies appear on the surface of cells, including circulating basophils. When the interaction allergen/IgE occurs, the cells presenting the IgE/allergen couple generates and releases chemical mediators, including histamine. This phenomenon leads in pathologic effects, such as vasodilatation, locally or systemically.

Usually, this milk hypersensitivity appears with the first attempt of food diversification when the infant is first presented to the cow's milk.

WO 01/97822 to Oy aboatech AB discloses the use of a probiotic bacteria, for example *Lactobacillus casei* ssp. *rhamnosus*, for a preparation of a composition useful for primary prevention of atopic diseases in an infant at high risk of atopic diseases. It is an attempt to prevent allergies in a specific population which is the high risks of allergy population.

WO 01/89541 to Compagnie Gervais Danone discloses the use of a Lactobacillus casei in a composition for oral administration to enhance immunity specific to pathogenic micro-organisms. It is particularly intended for pathogens affected respiratory system, and the composition can be a food or a food supplement.

However, a need exists for a mean of preventing atopic diseases such as cow's milk allergies and/or maintaining the oral tolerance acquired by the infant, either by himself or with the help of said aforementioned mean.

### Summary of the invention

Accordingly, the present invention refers to food products comprising at least a mean to promote the oral tolerance phenomenon, to food products comprising at least a mean to maintain the oral tolerance phenomenon, and to food products comprising means to promote and maintain the oral tolerance phenomenon.
The invention refers to food products which are not intended for allergic populations.

### Figures

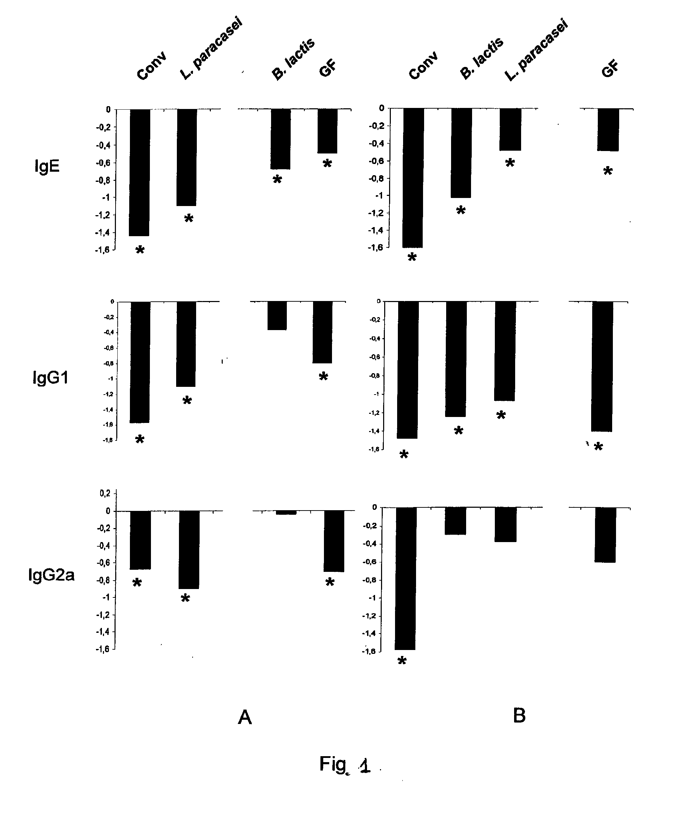

Fig 1 shows the decrease of BLG-specific antibody titers (IgE, IgG1 and IgG2a) in serum of mice (conventional (CV), monoassociated and germfree (GF)) killed 28 days (A) or 50 days (B) after oral feeding with whey proteins

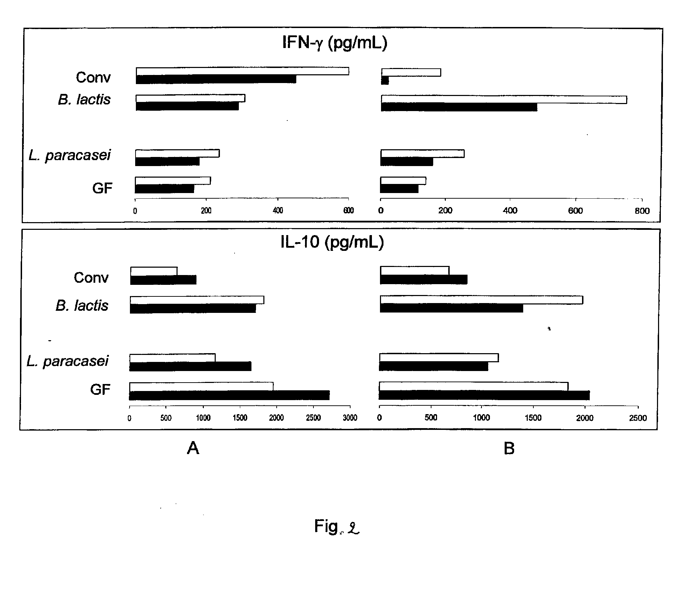

Fig 2 shows the production of IFN-γ, IL-10, IL-5 and IL-4 of spleen cells in vitro. Mice (Conventional (Conv), monoassociated and germfree (GF)) were orally fed whey proteins (black bars) or saline water (open bars) before being subcutaneously injected with 100 µg of BLG and 100 µg of OVA in Al(OH)₃ and sacrificed 28 days (A) or 50 days (B) after gavage. Mice sacrificed at day 50 received two additional injections at day 21 and 35..

### Detailed description of the invention

In the context of the present invention, the term "food product" is intended to encompass any consumable matter. Hence, it may be a product intended for the consumption by humans, but the term also encompasses products to be consumed by animals, for example pets, such as dogs, cats, rabbits, guinea pigs, mice, rats, birds (for example parrots), reptiles and fish (for example goldfish). However, the term also includes food to be consumed other domesticated animals, such as livestock, for example, cattle, horses, pigs, sheep, goats, buffaloes, camels, and the like. This term is also intended to include any infant formula, baby formula, infant and baby follow-up formula, and the like.

It has to be understood that when talking about cow's milk allergy or cow's milk hypersenstivity, we mean any food allergy or hypersensitivity, and more generally any atopic disease. Our argumentation is mostly developped around this allergy because cow's milk is in most cases the first food product encountered by infants; however, we do not limit the scope of the present patent application to this disease.

Surprisingly, we have found that the addition of probiotics to food products could be used as an adjuvant to promote oral tolerance, and to maintain it.

In a first aspect of the invention, there is provided a food product comprising at least a mean to promote the oral tolerance phenomenon to cow's milk proteins. Surprisingly, we have found that lactic acid bacteria strains belonging to the Lactobacillus genus, and in particular *Lactobacillus paracasei* strains are able to promote the induction of oral tolerance, and is particularly suitable to promote the oral tolerance to cow's milk proteins.
In a preferred embodiment of the invention, the Lactobacillus used is *Lactobacillus paracasei* CNCM I-2116. These microorganisms have been shown to exhibit inter alia the following properties: they are gram positive, catalase negative, NH₃ from arginine negative, and CO₂ production negative. They produce L(+) lactic acid and are capable to grow in the presence of bile salts in a concentration of up to 0.4%.
According to the invention, the lactic acid bacteria used can be added as an adjuvant or a supplement in particular to infant formulas used from birth to the introduction of antigens (such as cow's milk antigens) to the alimentation, in order to promote the oral tolerance to the said antigens. As the lactic acid bacteria will not be a dominant flora of the infant's intestine, large amounts of the microorganism should be incorporated to the formulae, for example 10⁵ to 10⁸ cfu/mL of reconstituted formulae or cfu/g of food product, more preferably 10⁵ to 10⁷, and in a preferred embodiment 10⁶ cfu/mL of reconsituted formula. Thus, the lactic acid bacteria will be ingested several times a day, leading to a quantity of this micro-organism in the gut microflora constantly sufficient to be effective for the purpose of the present invention.

The aforementioned lactic acid bacteria, belonging to the Lactobacillus genus, being in particular a *Lactobacillus paracasei* and in a preferred embodiment *Lactobacillus* *paracasei* CNCM I-2116 can also be incorporated in pet food products in order to promote oral tolerance. It can be incorporated in chunk products or croquettes preferably, but it can also be incorporated in humid products, such as cans, for example. It can also be added to liquid formulas for pets, such as milk for kittens or puppies, among others, and can as well be added to chew products for pets.
The supplementation of pet food by this micro-organism can be comprised, for example, between 10⁴ and 10⁸ cfu/g for non humid products, 10⁵ to 10⁸ cfu/g for humid products, and 10⁵ to 10⁸ cfu/g for liquid products, these amounts not being limitative.

In a second aspect of the invention, there is provided a food product comprising at least a mean to maintain the oral tolerance phenomenon, for example to cow's milk protein. Indeed, we have discovered that strains of the Bifidobacterium genus have a specific effect on the oral tolerance maintenance. Consequently, it is a purpose of the invention to add Bifidodacteria to foodstuffs as a supplement or an adjuvant, especially to populations wherein oral tolerance has already been induced. In particular, such population can be a breast-fed infant population: indeed, part of the allergens eaten by the mother are given to the child via the mother's milk; in this population, oral tolerance has been promoted via breast-feeding, and this tolerance can be maintained by giving to the infant an effective amount of Bifidobacteria having the aforementionned property.
It is well known that some infants, who do not develop cow's milk hypersensitivity when they first encounter cow's milk, nevertheless develop some food hypersensitivity when they encounter other food ingredients, for example at the age of 8 months. Although not wishing to be bound by theory, we believe that they have induced an oral tolerance which has not been maintained or not been maintained properly. Consequently, a purpose of the invention is to maintain an oral tolerance previously induced, by means of adding to the alimentation a Bifidobacteria having the ability to do so.

It might be useful to give the bifidus to the child or the infant at the beginning of the process.
In a preferred embodiment of the invention, the Bifidobacteria is a *Bifidobacterium lactis*, and can be in the best mode of realisation the well-known Bbl2 strain, ATCC 27536 which can be obtained from Hansen (Chr. Hansen A/S, 10-12 Boege Alle, P.O.Box 407, DK-2970 Hoersholm, Danemark). As the nomenclature has changed several time, Bbl2 can also befound in literature and in commercialised products as *B. bifidum and B.animalis.*
This microorganisms have been shown to exhibit inter alia the following properties: they are gram +, non mobitile and nonsporing rods, resistant to gastrointestinal acidity and bile salts. They are catalase negtive, and produce only L(+) lactic acid and not its D(-) isomer. They can utilize the following carbohydrates: ribose, saccharose, D-glucose, D-raffinose, maltose, melibiose, amygdalin and beta-gentobiose.

They can be added to any kind of infant, baby, or childhood food product, such as malted milk, infant formulae, follow up formulae, baby cereals and the like. Sometimes, diversification of the infant alimentation can occur as early of four months of life, and the bifidobacteria can be incorporated to the infant food given to children aged four months and older. As milk hypersensitivity in the majority disappears at the age of 2 to 3 years, supplementation of infant and baby food with Bifidobacterium to maintain oral tolerance can be done in every food product for infants and babies from 4 months to 3 years. But supplementation can also be realised in food products not specifically aimed for infant or baby nutrition and nevertheless being part of their alimentation, for example milk, yoghurt, curd, cheese, fermented milks, milk based fermented products, ice-creams, fermented cereal based products, or milk based products, among others.

The amount of microorganism added to the infant food product, baby food product or food product in general should be of from 10⁵ to 10⁸ cfu/mL of reconsituted formula or cfu/g of food product, preferably 10⁵ to 10⁷, and in a most preferred embodiment 10⁶. However these amounts should not be considered as limitative and should be adapted to the aimed population, for example based on the weight and age of the infant or baby, or specific populations such as population having specific diseases, for example infants, babies or children having gut diseases, acute diarrheas or inflammatory syndroms, among others.

The aforementioned Bifidobacteria, being in particular a *Bifidobacterium lactis* and in a preferred embodiment Bbl2 *Bifidobacterium lactis* ATCC 27536 can also be incorporated in pet food products in order to maintain oral tolerance. It can be incorporated in chunk products or croquettes preferably, but it can also be incorporated in humid products, such as cans, for example. It can also be added to liquid formulas for pets, such as milk for kittens or puppies, among others, and can as well be added to chew products for pets.
The supplementation of pet food by this micro-organism can be comprised, for example, between 10⁴ and 10⁸ cfu/g for non humid products, 10⁵ to 10⁸ cfu/g for humid products, and 10⁵ to 10⁸ cfu/g for liquid products, these amounts not being limitative.

In a third aspect of the invention, there is provided a food product comprising means to promote and maintain the oral tolerance phenomenon, for example to cow's milk proteins. Accordingly, a food product can comprise a combination of a Lactobacillus and a Bifidobacteria to both promote and maintain oral tolerance, in particular to cow's milk. In a preferred embodiment, the food product could comprise *Lactobacillus paracasei* and *Bifidobacterium lactis* to achieve the desired purpose. For example, the combination could comprise *Lactobacillus paracasei* CNCM I-2116 and *Bifidobacterium lactis* ATCC 27536. The means to promote and maintain oral tolerance can be a combination of one or several Lactobacilli with one or several Bifidobacteria. For example, it can be a combination of *Lactobacillus paracasei*, *Lactobacillus rhamnosus* and *Bifidobacterium lactis.* Depending on which population will ingerate the food supplemented with the microorganisms, the proportion of Lactobacilli compared to Bifidobacteria might vary. For example, for a population of infants which have not yet been in contact with cow's milk, the Lactobacilli:Bifidobacteria ratio could be comprised between 10:1 and 1:1. For a population of infants, babies or children having previously ingested cow's milk (or any other allergen or antogen), the Lactobacilli:Bifidobacteria ratio could be comprised between 1:10 and 1:1.

If more than one Bifidobacterium is incorporated to the food product, the ratio of one Bifidobacteria compared to the other can be from 0.01 to 99.9%, and the same applies if there is more than one Lactobacillus in the preparation.

The food product in which the combination of micro-organisms according to the invention is added can be any food product listed in the description of the first aspect of the invention and the second aspect of the invention, as well as any other food product, for example chocolate, chocolate powder, spreads, pastries, jellies, jams, biscuits, snacks, juices, dairy products, breakfast cereals, and more generally any food product eaten by infants, babies or children, as well as any food product eaten by pets.

The amount of microorganisms in the foodstuff according to the invention is preferably comprised between 10⁴ and 10⁸ cfu/g of food product or cfu/mL of reconstituted product when incorporated to humid or liquid products, and 10⁴ to 10⁹ cfu/g of food product when incorporated to non-humid or less humid products. The ratio Lactobacilli:Bifidocateria does not interfere on the quantity of microorganisms added into the food product.

Particularly, microorganisms of the invention, alone or in combination, help to prevent and maintain tolerance of ingested antigens. It is particularly suitable for food allergies, such as allergies to shellfish comprising shrimp, crayfish, lobster and crab, to peanuts, eggs, tree nuts (for example walnuts or cashew), soy, wheat, fish, and any other known food allergy. It is also suitable for other allergies, such as allergies to acarids, pollens or dusts, among others.

In a fourth aspect of the invention, there is provided a food product comprising probiotics to prevent from the risk of rejecting transplants. Such food product can comprise at least one member of the lactic acid bacteria family to induce oral tolerance, and/or at least one member of the Bifidobacteria genus to maintain an oral tolerance previously induced. The lactic acid bacteria strains belonging to the Lactobacillus genus are preferred, in particular *Lactobacillus paracasei*, and more particularly *Lactobacillus paracasei* CNCM I-2116. The Bifidobacteria used is preferably a *Bifidobacterium lactis*, and more particularly the Bb 12 strain ATCC 27536.
Indeed, although not wishing to be bound by theory, we believe that these two bacteria have the ability to specifically stimulate the immune system by promoting oral toleranceConsequently, the mechanism developed by the immune system together with these bacterias, alone or in combination, moving away allergies is not specific to cow's milk allergy or hypersensitivity, and this mechanism can help patients in need of a transplantation or having been transplanted not to reject their transplant. An aspect of the present invention is so to prevent the risk of graft versus host diseases appearance by helping to the induction and the maintain of the oral tolerance to the graft, prior the transplatation and thereafter, in order to help patients to support transplantation, from an immunological point of view, by acquiring oral tolerance to the antigen that will be transplanted..

The microorganisms added to the food products according to the invention are preferably alive but can also be dead ar can be inactivated for example by lyophilisation. Indeed, the external membrane of the dead bacterias would seem to activate the immune system, at least partly, in the same way than the alive bacterias. Consequently, in any embodiment of the invention, the bacterias or combination of bacterias can be achieved with dead organisms. In this case, the amount of microorganisms incorporated to the food product is preferably enhanced compared to the amounts given above, from 5% to 250%.

### Examples

The following examples are illustrative of some of the products and methods of making the same falling within the scope of the present invention. They are not to be considered in any way limitative of the invention. Changes and modifications can be made with respect to the invention. That is, the skilled person will recognise many variations in these examples to cover a wide range of formulas, ingredients, processing, and mixtures to rationally adjust the naturally occurring levels of the compounds of the invention for a variety of applications.

### Example 1: induction of tolerance

3 groups of 20 germ-free female mice C3H/HeJ 3 to 5 weeks old and one group of conventional female mice BALB/c 3 weeks old were used in this experiment. Two probiotic strains were used in the study: one isolated from faeces of healthy babies was from the Nestlé Culture Collection (Lausanne, Switzerland): *Lactobacillus paracasei* NCC 2461 (CNCM I-2116) and one was purchased from Chris Hansen (France) and is of human origin: *Bifidobacterium lactis* Bbl2 NCC 362.
The germ free mice were inoculated by the oral route with an intragastric tube with 0.3 mL of a 24 hours bacterial culture containing around 5.10⁸ cfu/mL of one out of the *L. paracasei* or *B. lactis* strains. Oral induction was induced two weeks after bacterial feeding. In each group, 10 mice were given oral administration of 3 mg/g body weight whey proteins to induce oral tolerance. Whey proteins were obtained by ultrafiltration of acid whey, and the protein content was 80%, approximately 62% of which was β-lactoglobulin. The remaining 10 mice orally received a single feeding of saline water as negative control (non-tolerized mice).
Five days later, all mice were immunised subcutaneously with 100 µm β-lactoglobulin (BLG) 3 times crystallised and 100 µg ovalbumin grade V (OVA). Mice were sacrificed 28 or 50 days after gavage to assess induction and maintenance of tolerance, respectively. At each sacrifice, 5 whey-protein fed and 5 water-fed mice were killed. Mice sacrificed at 50 days received two additional subcutaneous injections of BLG and OVA at day 21 and 35.
The amount of BLG-specific IgE and BLG-specific IgG1 and IgG2a levels in serum were determined by ELISA.

The induction of oral tolerance in conventional, germfree and monoassiociated mice were assessed in mice sacrificed 28 days after oral administration of whey proteins and immunised with BLG: see figure 1A. The results clearly showed that whey proteins feeding significantly suppressed (P<0.05) the BLG-specific IgE, IgG1 and IgG2a antiboby responses in conventional, germfree and *L.paracasei*-associated mice indicating a Th1- and Th2- type humoral responses suppression. In mice associated with *B. lactis*, only anti-BLG IgE response was significantly suppressed: anti-BLG IgG1 response was suppressed but not significantly (P<0.2). Hence, only the Th2-type humoral responses were sensitive to tolerance in this group of mice.

Induction of tolerance in terms of spleen cells proliferation and cytokine production: to further confirm that tolerance induction is modulated by probiotics, spleen cells were cultured in vitro and both cell proliferation (table 1) and cytokine profiles in culture supernatants were examined (figure 2A)

**Table 1**

| *Proliferative response of BLG- or PHA-activated splenocytes from conventional (Conv), monoassociated and germfree (GF) mice killed 28 days or 50 days after oral feeding with whey proteins.* | | | | | | | | | | |
|---|---|---|---|---|---|---|---|---|---|---|
| Proliferative index | | | | | | | | | | |
| | Proliferation (cpm) | | | | | | Stimulation index | | | |
| | | Day 28 | Day 50 | | | | Day 28 | | Day 50 | |
| | Ag(-) | BLG | PHA | Ag(-) | BLG | PHA | BLG | PHA | BLG | PHA |
| Conv | 212 | 3.174 | 70.541 | 311 | 7.214 | 86.268 | 15 | 332 | 23 | 277 |
| B.lactis | 273 | 13.355 | 30.328 | 401 | 27.403 | 53.806 | 49 | 111 | 61 | 134 |
| L.paracasei | 361 | 5.881 | 45.341 | 763 | 20.919 | 78.489 | 16 | 126 | 27 | 102 |
| GF | 281 | 8.302 | 58.284 | 256 | 33.744 | 40.64 | 29 | 207 | 131 | 158 |

### Example 2: maintenance of oral tolerance

The experiment is the continuation of example 1.
The maintenance of specific antibody response suppression in tolerized mice was monitored for a 7-week period after whey proteins feeding (figure 1). BLG-specific IgE, IgG1 and IgG2a responses were maintained suppressed in conventional mice (p<0.01). The suppression of anti-BLG IgG2a titer in germ free and *L. paracasei*-associated mice was not maintained, whereas both specific IgE and IgG1 levels were. A continued suppression of BLG-specific IgE responses and a significant decrease anti-BLG IgG1 titer were observed in mice colonised with *B. lactis.* As result, Th1- and Th2- type humoral responses were maintained suppressed in conventional mice, while only Th1-type antibody titers were maintained abrogated in germfree, *B. lactis* and *L. paracasei*-associated mice.
IFN-γ productions by spleen cells in mice sacrificed 50 days after oral tolerance induction were similar to those obtained after 28 days, except for germfree and *B. lactis*-associated mice for which the decrease became not significant and significant, respectively (Figure 2B). The Th1-cell activity also remained suppressed in all groups of mice (Figure 2B). In contrast, the high production of IL-10 was maintained only in conventional mice given whey proteins (Figure 2B). Nevertheless, spleen cells from germfree and *B. lactis*-associated mice have been found to be sensitive to long-term tolerance persistence clearly shown by high stimulation indexes reaching 131, 61 and 38, respectively, in presence of BLG (Table 1). Lower stimulation indexes were obtained in conventional mice and mice colonized with *L. paracasei* in presence of BLG, while cells from all the groups of mice responded well to PHA (Table 1).

### Example 3: infant formulae promoting and inducing oral tolerance

We prepare an infant formula by mixing together the following ingredients in the indicated proportions. The final product comprises 519 kcal for 100 g, and is in powder form.

| | | | |
|---|---|---|---|
| Fat | | | 27.7 g |
| | Fat from milk | | 0.7 g |
| | Mixture of fats (150) | | 26.8 g |
| | Lecithin | | 0.2 g |
| | | Linoleic acid | 4.1 g |
| | | α-linolenic acid | 525 mg |
| Proteins | | | 9.5 g |
| Free carbohydrates | | | 57.9 g |
| | Lactose | | 57.9 g |
| Minerals (ashes) | | | 1.9 g |
| | Sodium | | 120 mg |
| | Potassium | | 460 mg |
| | Chlorure | | 330 mg |
| | Calcium | | 320 mg |
| | Phosphorus | | 160 mg |
| | Magnesium | | 36 mg |
| | Manganese | | 40 µg |
| | Selenium | | 10.4 µg |
| Total solids | | | 97.0 g |
| Humidity | | | 3.0 g |

We then add to the above-prepared formulae 10⁶ per 100g of *Lactobacillus paracasei*, and 10² of *Bifidobacterium lactis.*

### Example 4: cat food promoting and inducing oral tolerance

A feed mixture is made up of about 58% by weight of corn, about 6% by weight of corn gluten, about 23% by weight of chicken meal, salts, vitamins and minerals making up the remainder.

The feed mixture is fed into a preconditioner and moistened. The moistened feed is then fed into an extruder-cooker and gelatinised. The gelatinised matrix leaving the extruder is forced through a die and extruded. The extrudate is cut into pieces suitable for feeding to cats, dried at about 110°C for about 20 minutes, and cooled to form pellets. At this point, a lyophilised powder of one or more strains of the following *species* is provided for application to the pellets: *Lactobacillus paracasei* and *Bifidobacterium lactis*. Sufficient powder is thus provided so that the corresponding dietary intake amount for the cat is from about 10⁷-10⁹ cfu / day . Some of the powder is mixed into a first mass of pellets and bagged. A second quantity of the powder is measured out and mixed with a lipid carrier which is then sprayed on to a second mass of pellets. The pellets are bagged after the coating has dried sufficiently at 50-60°C for some minutes.

## Claims

1. Food product comprising at least a mean to promote oral tolerance, such mean consisting in the addition of a Lactobacillus strain.

2. Food product according to claim 1 wherein the Lactobacillus is a *Lactobacillus paracasei.*

3. Food product according to claim 1 wherein the Lactobacillus is *Lactobacillus paracasei* CNCM I-2116.

4. Food product according to one of claims 1 to 3 wherein the added bacterias are inactivated or dead.

5. Food product according to one of claims 1 to 4 wherein the food product is not intended for allergic populations.

6. Food product according to one of claims 1 to 5 wherein the food product is an infant formula.

7. Food product according to one of claims 1 to 6 wherein the bacterias are present in the food product in an amount of 10⁵ to 10⁸ cfu/mL of reconstituted formulae or cfu/g of food product, more preferably 10⁵ to 10⁷, and in a preferred embodiment 10⁶ cfu/mL of reconsituted formula.

8. Food product according to one of claims 1 to 5 wherein the food product is a pet food.

9. Food product according to claim 8 wherein the bacterias are present in an amount of 10⁴ and 10⁸ cfu/g for non humid products, 10⁵ to 10⁸ cfu/g for humid products, and 10⁵ to 10⁸ cfu/g for liquid products.

10. Food product comprising at least a mean to maintain oral tolerance, such mean consisting in the addition of a Bifidobacterium strain.

11. Food product according to claim 10 wherein the Bifidobacterium is a *Bifidobacterium lactis.*

12. Food product according to claim 10 wherein the Bifidobacterium is *Bifidobacterium lactis* ATCC 27536.

13. Food product according to one of claims 10 to 12 wherein the added bacterias are inactivated or dead.

14. Food product according to one of claims 10 to 13 wherein the food product is not intended for allergic populations.

15. Food product according to one of claims 10 to 14 wherein the food product is an infant formula.

16. Food product according to one of claims 10 to 15 wherein the bacterias are present in the food product in an amount of 10⁵ to 10⁸ cfu/mL of reconsituted formula or cfu/g of food product, preferably 10⁵ to 10⁷, and in a most preferred embodiment 10⁶.

17. Food product according to one of claims 10 to 14 wherein the food product is a pet food.

18. Food product according to claim 17 wherein the bacterias are present in an amount of 10⁴ and 10⁸ cfu/g for non humid products, 10⁵ to 10⁸ cfu/g for humid products, and 10⁵ to 10⁸ cfu/g for liquid products.

19. Food product comprising at least a mean to promote oral tolerance and a mean to maintain oral tolerance.

20. Food product according to claim 19 wherein the mean to promote and the mean to maintain the oral tolerance are the addition of at least two lactic acid bacteria strains.

21. Food product according to claim 20 wherein the lactic acid bacteria to promote oral tolerance is a Lactobacillus and the lactic acid bacteria to maintain oral tolerance is a Bifidobacteria.

22. Food product according to claim 21 wherein the Lactobacillus is *a Lactobacillus paracasei* and the Bifidobacterium is a *Bifidobacterium lactis.*

23. Food product according to claim 22 wherein the *Lactobacillus paracasei* is *Lactobacillus paracasei* CNCM I-2116 and the *Bifidobacterium lactis* is *Bifidobacterium lactis* ATCC 27536.

24. Food product according to claim 21 wherein there is at least one Lactobacillus and/or at least one Bifidobacteria.

25. Food product according to one of claims 19 to 24 wherein the food product is not intended for allergic populations.

26. Food product according to one of claims 21 to 24 wherein the food product is an infant formula.

27. Food product according to claim 26 wherein the Lactobacilli:Bifidobacteria ratio is comprised between 10:1 and 1:1 in a population of infants which have not been in contact with each new food antigen.

28. Food product according to claim 26 wherein the Lactobacilli:Bifidobacteria ratio is comprised between 1:10 and 1:1 in a population of infants, babies or children which have been in contact with food antigens.

29. Food product according to one of claims 19 to 25 wherein the food product is a pet food.

30. Food product according to one of claims 19 to 29 wherein the amount of microorganisms in the foodstuff according to the invention is preferably comprised between 10⁴ and 10⁸ cfu/g of food product or cfu/mL of reconstituted product when incorporated to humid or liquid products, and 10⁴ to 10⁹ cfu/g of food product when incorporated to non-humid or less humid products.

31. Food product comprising probiotics to prevent allergies and/or to decrease the risk of rejecting transplants.

32. Food product according to claim 31 comprising at least one member of the lactic acid bacteria family to induce oral tolerance, and/or at least one member of the lactic acid bacteria family to maintain an oral tolerance previously induced.

33. Food product according to claim 32 wherein the lactic acid bacteria inducing oral tolerance belongs to the Lactobacillus genus, is preferably *Lactobacillus paracasei*, is in the preferred embodiment *Lactobacillus paracasei* CNCM I-2116.

34. Food product according to claim 32 wherein the lactic acid bacteria maintaining oral tolerance belongs to the Bifidobacteria genus, is preferably a *Bifidobacterium lactis*, is in a preferred embodiment Bb 12 strain ATCC 27536.

35. Food product according to one of claims 31 to 35 wherein the allergy is a food allergy.
